# EUROPÄISCHE PATENTSCHRIFT

(11) **EP 3 911 282 B1**
(45) Veröffentlichungstag und Bekanntmachung des Hinweises auf die Patenterteilung: **12.04.2023**
(21) Anmeldenummer: 20701023.2
(22) Anmeldetag: 16.01.2020
(51) Int. Cl.: A61F 2/60, A61F 2/64, A61F 2/68, A61F 2/72

(54) **VERFAHREN ZUR STEUERUNG EINER ORTHETISCHEN ODER PROTHETISCHEN EINRICHTUNG UND ORTHETISCHE ODER PROTHETISCHE EINRICHTUNG**
METHOD FOR CONTROLLING AN ORTHOTIC OR PROSTHETIC DEVICE AND ORTHOTIC OR PROSTHETIC DEVICE
PROCÉDÉ POUR LA COMMANDE D'UN DISPOSITIF ORTHÉTIQUE OU PROTHÉTIQUE ET DISPOSITIF ORTHÉTIQUE OU PROTHÉTIQUE

(30) Priorität: 17.01.2019 DE 102019101143
(43) Veröffentlichungstag der Anmeldung: 24.11.2021
(73) Patentinhaber: Otto Bock Healthcare Products GmbH, 1110 Wien (AT)
(72) Erfinder: SEIFERT, Dirk, 1070 Wien (AT); MEJIA NINO, Juan-Pablo, 2340 Mödling (AT)
(74) Vertreter: Gramm, Lins & Partner Patent- und Rechtsanwälte PartGmbB
(86) Internationale Anmeldenummer: PCT/EP2020/051003
(87) Internationale Veröffentlichungsnummer: WO 2020/148373

(56) Entgegenhaltungen:
- DE-A1- 19 521 464
- DE-A1-102015 001 967
- DE-B3-102017 119 490

## Beschreibung

Die Erfindung betrifft ein Verfahren zur Steuerung einer orthetischen oder prothetischen Einrichtung wie in den Ansprüchen definiert. Die Erfindung betrifft ebenso eine solche orthetische oder prothetische Einrichtung wie in den Ansprüchen definiert.

Prothetische Einrichtungen oder Orthesen haben die Aufgabe, die Bewegung einer vorhandenen Gliedmaße zu führen oder zu unterstützen, bzw. eine Gliedmaße abzustützen und zu unterstützen. Exoskelette sind ebenfalls orthetische Einrichtungen. Orthetische Einrichtungen für die untere Extremität sind in unterschiedlichen Ausführungsformen bekannt, sogenannte KAFO (knee ankle foot orthosis) stützen sowohl den Fuß als auch das Knöchelgelenk und das Kniegelenk ab. Der Fuß wird in der Regel auf einer Fußplatte aufgesetzt, eine oder mehrere Unterschenkelschienen erstrecken sich parallel zu dem Unterschenkel und ein Orthesenkniegelenk ist ungefähr im Bereich der natürlichen Knieachse vorgesehen. An einer oder mehreren Oberschenkelschienen sind Befestigungseinrichtungen zum Festlegen der Orthese an dem Oberschenkel angebracht. Ebenso können Befestigungseinrichtungen an dem Unterschenkel oder der Fußplatte vorgesehen sein, um die Orthese an dem jeweils zu versorgenden Bein festlegen zu können. Bezüglich des Orthesenkniegelenkes ist die Oberschenkelschiene eine proximale Komponente und die Unterschenkelschiene eine distale Komponente, bezüglich eines Orthesenknöchelgelenkes ist das Fußteil die distale Komponente und die Unterschenkelschiene oder die Unterschenkelschienen die proximale Komponente. Zwischen den jeweiligen Komponenten können Dämpfer oder Antriebe als Aktuatoren angeordnet sein, um Bewegungsabläufe zu beeinflussen und Bewegungswiderstände zu verändern. Es kann entweder ein erhöhter Bewegungswiderstand zum Dämpfen einer Bewegung oder ein verringerter Bewegungswiderstand durch Verringerung einer Dämpfung oder Unterstützung mittels eines oder mehrerer motorischer oder kraftspeichergetriebener Antriebe bereitgestellt werden. Entsprechendes gilt auch für orthetische Einrichtungen wie Orthesen und Exoskelette für die obere Extremität.

Prothesen ersetzen eine nicht vorhandene Gliedmaße und können ein künstliches Gelenk aufweisen. Prothesen der unteren Extremität können beispielsweise ein Prothesenkniegelenk aufweisen, das über ein Unterschenkelrohr als Unterschenkelteil mit einem Prothesenfuß als Fußteil verbunden ist. Zwischen dem Prothesenfuß und dem Unterschenkelrohr kann ein Prothesenknöchelgelenk ausgebildet sein. Das Prothesenkniegelenk weist in der Regel ein Oberteil und ein Unterteil auf, die entweder über eine monozentrische oder polyzentrische Gelenkeinrichtung schwenkbar aneinander gelagert sind, so dass das Oberteil relativ zu dem Unterteil um eine Schwenkachse verschwenkbar ist. An dem Oberteil ist zumindest eine Befestigungseinrichtung angeordnet, um die Prothese der unteren Extremität an einem Anwender festlegen zu können, beispielsweise für einen Oberschenkelschaft oder in Gestalt eines Oberschenkelschaftes. Bezüglich des Prothesenknöchelgelenkes ist das Unterschenkelrohr das Oberteil und der Prothesenfuß das Unterteil. Zwischen dem jeweiligen Oberteil und dem Unterteil oder der proximalen Komponente und der distalen Komponente kann jeweils ein Aktuator angeordnet sein, um den jeweiligen Bewegungswiderstand hinsichtlich einer Flexion und/oder Extension zu verändern. Wie im Zusammenhang mit einer Orthese ausgeführt, kann der Bewegungswiderstand über den Aktuator vergrößert oder verringert werden, beispielsweise über passive, verstellbare Dämpfer oder Antriebe wie Elektromotoren oder kraftspeichergetriebene Antriebe. Neben Prothesen für untere Extremitäten sind entsprechende Prothesen auch für die oberen Extremitäten bekannt. Die oben beschriebenen Merkmale einer Orthese, Prothesen oder Exoskelettes können auch an die erfindungsgemäßen orthetischen und prothetischen Einrichtungen vorhanden sein.

Aus der DE 10 2008 008 284 A1 ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssensor, ein Neigungssensor und/oder ein Kraftsensor. In Abhängigkeit von den Sensordaten wird die Position eines Extensionsanschlages ermittelt und entsprechend eingestellt.

Aus der DE 10 2009 052 887 A1 ist ein Verfahren zur Steuerung eines orthetischen oder prothetischen Gelenkes mit einer Widerstandseinrichtung und Sensoren bekannt, wobei über die Sensoren während der Benutzung des Gelenkes Zustandsinformationen bereitgestellt werden. Die Sensoren erfassen Momente oder Kräfte, wobei die Sensordaten von zumindest zwei der ermittelten Größen durch eine mathematische Operation miteinander verknüpft werden und dadurch eine Hilfsvariable errechnet wird, die der Steuerung des Beugewiderstandes und/oder des Streckwiderstandes zugrunde gelegt wird.

Darüber hinaus ist aus dem Stand der Technik bekannt, über Elektroden Muskelkontraktionen eines Anwenders zu erfassen, beispielsweise über Oberflächenelektroden oder über implantierte Elektroden, um auf der Grundlage von erfassten Muskelkontraktionen Steuerungsbefehle zu generieren, z.B. um Antriebe zu aktivieren oder zu deaktivieren. Solche auf myoelektrischen Signalen basierende Steuerungen werden insbesondere bei Prothesen der oberen Extremität eingesetzt, um komplexe Bewegungen der Prothesenhand zu steuern.

Orthetische oder prothetische Einrichtungen der unteren Extremität werden überwiegend über die Bewegungen und Belastungen des Körpers bzw. der orthetischen oder prothetischen Einrichtung beeinflusst, die auftretenden Beschleunigungen oder Kräfte oder Zustände werden von Sensoren erfasst und den Sensorsignalen basierend wird ein bestimmtes Dämpfungsverhalten eingestellt. Eine willentliche Beeinflussung ist nur über eine Veränderung der Bewegungen oder Systembelastungen möglich. Bei aktiven, angetriebenen orthetischen oder prothetischen Einrichtungen ist es bekannt, über eine proportionale Zuordnung von Muskelaktivität zum Antrieb eine Verlagerung von Komponenten zueinander zu bewirken, beispielsweise eine Hand zu öffnen oder zu schließen oder über eine Mustererkennung bestimmte Programme anzusteuern. Kontraktionssignale werden erfasst, um zwischen verschiedenen Betriebsmodi hin- und herzuschalten.

Die DE 10 2015 001 967 A1 beschreibt eine steuerbare orthopädietechnische Einrichtung, die z.B. mittels Erfassung von Muskelkontraktionen gesteuert wird. Die DE 10 2017 119 490 B1 betrifft ein Verfahren zum Überprüfen der Funktionsfähigkeit eines Prothesensystems. Beide Dokumente befassen sich nur mit der Erfassung von Muskelkontraktionen.

Die DE 195 21 464 A1 betrifft ein Verfahren zur Steuerung einer Kniebremse eines Prothesen-Kniegelenks, wobei an mehreren, dem Hüftgelenk zugeordneten Muskeln jeweils EMG-Sensoren angeordnet sein können. Die Kontraktion dieser Muskeln wird zur Steuerung der Kniebremse herangezogen.

Aufgabe der vorliegenden Erfindung ist es, für Anwender eine orthetische oder prothetische Einrichtung und ein Verfahren zu deren Steuerung bereitzustellen, die eine erhöhte Sicherheit für den Nutzer bereitstellen, bei einer gleichzeitig einfachen Implementierung mit einem möglichst geringen Kostenaufwand.

Erfindungsgemäß wird diese Aufgabe durch ein Verfahren und eine orthetische oder prothetische Einrichtung mit den Merkmalen der unabhängigen Ansprüche gelöst, vorteilhafte Ausgestaltungen und Weiterbildungen der Erfindung sind in den Unteransprüchen, der Beschreibung sowie den Figuren offenbart.

Das Verfahren zur Steuerung einer orthetischen oder prothetischen Einrichtung, die an einem Körper eines Anwenders anlegbar und daran festlegbar ist, mit einer Gelenkeinrichtung mit einer proximalen Komponente und einer distalen Komponente, die um eine Schwenkachse schwenkbar aneinander gelagert sind, zumindest einen verstellbaren Aktuator, der zwischen der proximalen Komponente und der distalen Komponente angeordnet ist und über die ein Bewegungswiderstand gegen eine Verschwenkung der proximalen Komponente relativ zu der distalen Komponente einstellbar ist, zumindest einer Erfassungseinrichtung zur Erfassung von Muskelkontraktionen sowie einer Steuerungseinrichtung, die mit der Erfassungseinrichtung und dem Aktuator gekoppelt ist, Signale von der Erfassungseinrichtung verarbeitet und in Abhängigkeit von den Signalen den Aktuator verstellt, sieht vor, dass die Erfassungseinrichtung zur Erfassung von Muskelkokontraktionen eingerichtet ist und an einer Gliedmaße des Anwenders angeordnet und mit der Steuerungseinrichtung gekoppelt wird, dass zumindest eine Muskelkokontraktion von der Erfassungseinrichtung detektiert wird, dass ermittelt wird, ob eine Kokontraktion vorliegt und dass der Bewegungswiderstand des Aktuators in Abhängigkeit von der detektierten Muskelkokontraktion verändert wird. Eine Muskelkokontraktion ist das Anspannen von Agonisten und Antagonisten zur Gelenkstabilisierung oder das gleichzeitige Anspannen zweier entgegengesetzt wirkender Muskeln oder Muskelgruppen. Eine Muskelkokontraktion liegt beispielsweise vor, wenn der Armbizeps und der Trizeps gleichzeitig angespannt werden, ohne dass sich der Unterarm relativ zu dem Oberarm bewegt oder aber je nach Kontraktionsniveau langsamer als ohne Anspannung des Antagonisten bewegt. Entsprechendes gilt für die den Unterschenkel streckende und beugende Muskulatur. Werden einander entgegengesetzt wirkende Muskeln oder Muskelgruppen gleichzeitig angespannt, spricht man von Muskelkokontraktionen. Muskelkokontraktionen werden dazu eingesetzt, um Gelenke oder Gliedmaße zu stabilisieren, um bei einer Ausführung von Bewegungen ein Höchstmaß von Kontrolle zu haben oder um Bewegungen abzustoppen. Muskelkokontraktionen erfolgen bewusst oder unbewusst. Eine unbewusste Kokontraktion tritt beispielsweise als Reflex bei plötzlichen Störungen, wie auftretender Glätte, rutschigem Boden, Schrecksituationen wie Explosionen, Unfällen oder ähnlichem oder aber auch zur Vorbereitung auf möglicherweise gefährliche Situationen auf, wie das Entlanggehen an einem Abgrund oder über eine Brücke. Über eine Muskelkokontraktion wird die Grundspannung in dem muskulären System erhöht. Wird über die Erfassungseinrichtung eine entsprechende Muskelkokontraktion erfasst oder detektiert, werden also beispielsweise das gleichzeitige Anspannen von Armbizeps und Armtrizeps oder beispielsweise des musculus biceps femoris und des musculus quadriceps femoris von der Erfassungseinrichtung erfasst, werden von der Erfassungseinrichtung entsprechende Signale an die Steuerungseinrichtung übermittelt, die dann in Abhängigkeit von den detektieren Muskelkokontraktionen den Bewegungswiderstand des Aktuators verändert. Die Veränderung des Bewegungswiderstandes kann beispielsweise durch das Verstellen von Ventilen in einem Hydraulikdämpfer oder einem Pneumatikdämpfer erfolgen. Ebenso können Bremsen gelöst oder aktiviert werden oder andere Widerstände vergrößert oder verringert werden, beispielsweise über elektrische Antriebe die zugeschaltet oder gesperrt oder in einer Verlagerung der proximalen Komponente relativ zu der distalen Komponente entgegenwirkt. Bevorzugt bleibt die Grundeinstellung des Aktuators unverändert, das heißt, dass die einmal eingestellte Widerstandskurve oder ein Widerstandsverlauf über eine bestimmte Bewegung oder eine bestimmte Belastung beibehalten wird, also dass grundsätzliche Widerstandsverhalten über die Bewegung unverändert bleibt. Bei ausgewählten Muskeln, insbesondere bei Muskeln, die für die Ausführung der jeweiligen Bewegung zuständig sind oder zuständig wären, wenn die Gliedmaße noch vorhanden wäre, wird die Muskelaktivität über die Erfassungseinrichtung erfasst. Wird dann eine willkürliche oder unwillkürliche Muskelkokontraktion detektiert, wird auf eine Sondersituation geschlossen, die eine Veränderung des Bewegungswiderstandes erforderlich macht. Anders als bei Prothesensteuerungen der oberen Extremität werden Muskelkokontraktionen nicht zum Umschalten und zum Einschalten eines neuen Betriebsmodus eingestellt, beispielsweise um von dem Betriebsmodus des Handöffnens auf den Betriebsmodus des Handschließens umzuschalten, vielmehr werden die gleichen Bewegungen weiterhin zugelassen, jedoch auf einem anderen Widerstandsniveau oder der Widerstand wird soweit erhöht, dass eine Weiterbewegung des Gelenkes bei üblichen Belastungsszenarien nicht mehr möglich ist. Es handelt sich somit um eine Variation eines originären Steuerungsverhaltens, basierend auf der erfassten Kokontraktion.

In einer Ausführungsform der Erfindung werden die Dauer und/oder die Intensität der Muskelkokontraktion oder der Muskelkokontraktionen erfasst und in Abhängigkeit von der Dauer und/oder der Intensität der Muskelkokontraktion oder Muskelkokontraktionen wird der Bewegungswiderstand verändert. Beispielsweise wird bei einer länger andauernden Muskelkokontraktion der Bewegungswiderstand über einen längeren Zeitraum verändert. Ebenso kann bei einer hohen Muskelkokontraktionsintensität eine Vergrößerung der Veränderungsamplitude verglichen mit einer geringeren Muskelkokontraktionsintensität ausgeführt werden, um den Bewegungswiderstand angepasst zu verändern. Beispielsweise kann der Bewegungswiderstand bei der Detektion einer Muskelkokontraktion vergrößert werden, was dem Effekt der Muskelkokontraktion bei einer gesunden Gliedmaße entspricht. Bemerkt beispielsweise der Nutzer der orthetischen oder prothetischen Einrichtung vor sich eine Veränderung der Beschaffenheit des Untergrundes, beispielsweise eine feuchte Stelle auf einem glatten Boden, wird durch die Muskelkokontraktion der Bewegungswiderstand beispielsweise in einem Prothesenkniegelenk oder Orthesenkniegelenk vergrößert, wodurch der Anwender ein erhöhtes Sicherheitsgefühl erhält, da die Gelenkeinrichtung besser von der übrigen Muskulatur kontrolliert und orientiert werden kann. Bei einer Prothese erfolgt die Kontrolle dann über den Oberschenkelstumpf. Bei aktiven orthetischen oder prothetischen Einrichtungen, also Einrichtungen mit motorischen oder anderweitigen Antrieben, können die eine Bewegung unterstützenden Kräfte verringert, Widerstände in den Antrieben vergrößert oder Antriebe gegenläufig aktiviert werden, um ein Verschwenken der distalen und proximalen Komponenten zueinander zu verhindern oder zu behindern.

Bei zunehmender Muskelkokontraktionsintensität und/oder Muskelkokontraktionsdauer kann der Bewegungswiderstand zunehmend vergrößert werden, so dass durch die Erfassung des zeitlichen Verlaufes der Muskelkokontraktionsintensität und/oder Muskelkokontraktionsdauer eine proportionale Veränderung des Bewegungswiderstandes erfolgen kann. Steigert sich die Intensität über einen Kokontraktionszeitraum, wird die Veränderung vergrößert. Ebenso ist es möglich, dass unabhängig von der Intensität der Kokontraktion der Bewegungswiderstand bei Vorliegen einer Kokontraktion solange vergrößert wird, wie die Kontraktion andauert, also dass bei einer längeren Kokontraktionsdauer eine größere Veränderung als bei einer kurzen Kokontraktionsdauer ausgeführt wird. Alternativ oder ergänzend kann am Ende einer Kokontraktion und/oder bei Detektion einer anderen Kokontraktion und/oder durch einen aktiven Auslöser und/oder einen Sprachbefehl der Widerstand verringert werden. Alle Veränderungsmethoden können alleine oder auch gemeinsam oder in einer beliebigen Kombination angewendet werden.

Eine Weiterbildung der Erfindung sieht vor, dass der Widerstand zunächst sehr schnell erhöht wird, also dass ein steiler Anstieg der Widerstandskraft erfolgt, wobei die Verringerung des Widerstandes langsam, zumindest langsamer als der Anstieg erfolgt, also der Abfall der Widerstandskraft weniger steil ausgeführt wird.

Die Veränderung des Bewegungswiderstandes kann einem voreingestellten Steuerungsprogramm überlagert werden. Moderne Orthesen oder Prothesen weisen häufig Aktuatoren auf, die sensorgesteuert verschiedene Bewegungswiderstände bereitstellen, so dass beispielsweise in einem Gangzyklus unterschiedliche Flexions- und Extensionswiderstände in den jeweiligen Gangphasen eingestellt werden. Durch Lagesensoren, Kraftsensoren, Momentensensoren, Winkelsensoren und Beschleunigungssensoren sowie anderer Sensoren ist es möglich, den gegenwärtigen Zustand der Prothese, Orthese oder eines Exoskelettes zu erfassen und ein an den Zustand oder das Bewegungsverhalten angepasstes Widerstandsverhalten bereitzustellen. Dieses Steuerprogramm kann auch bei der Detektion einer Muskelkokontraktion grundsätzlich beibehalten werden, das heißt, dass das bereits bestehende Dämpfungskonzept oder Antriebskonzept basierend auf den entsprechenden Sensoren grundsätzlich unverändert bleiben kann. Lediglich das Widerstandsniveau oder die Dauer unterschiedlicher Widerstände werden für die Dauer der Muskelkokontraktion beeinflusst. Die Verläufe der Widerstände über die Zeit bei dem normalen Steuerungsprogramm können in der Amplitude und/oder über das zeitliche Ausmaß durch die Veränderung des Bewegungswiderstandes durch die detektierte Muskelkokontraktion verändert werden. Dadurch werden insgesamt die Stabilität der jeweiligen Gelenkeinrichtung und damit auch die Stabilität der orthetischen oder prothetischen Einrichtung vergrößert. Bei Wegfall der Muskelkokontraktion wird wieder das voreingestellte Standardprogramm aktiviert.

Grundsätzlich ist es auch möglich, dass die Veränderung des Bewegungswiderstandes auch bei Aktuatoren angewendet wird, die nicht über ein anderweitig sensorgesteuertes Steuerungsverfahren verfügen, sondern beispielsweise fest eingestellt an dem jeweiligen Anwender ein bestimmtes Dämpfungsverhalten oder Bewegungswiderstandsverhalten aufweisen.

Die Veränderung des Bewegungswiderstandes kann die Größe des Bewegungswiderstandes, also die Amplitude des Bewegungswiderstandes, und/oder die Dauer des Bewegungswiderstandes, also den zeitlichen Verlauf über den Bewegungszyklus, beeinflussen.

Die Muskelkontraktionen und damit auch die Muskelkokontraktionen werden von der Erfassungseinrichtung in einem Ausführungsbeispiel als myoelektrische Signale, Drucksignale, induktiv erzeugte Signale und/oder opto-elektronisch erzeugte Signale erfasst und der Steuerungseinrichtung übermittelt. Die Steuerungseinrichtung ist als eine Datenverarbeitungseinrichtung ausgebildet und weist einen Prozessor oder einen Computer auf, der elektrische Signale verarbeitet. Die Signale werden von Sensoren oder Detektoren erzeugt, die unterschiedliche physikalische, chemische oder biologische Effekte ausnutzen. Die Erfassungseinrichtung kann beispielsweise Oberflächenelektroden zur Erfassung myoelektrischer Signale aufweisen. Myoelektrische Signale entstehen bei der Kontraktion von Muskeln. Der Vorteil von Oberflächenelektroden ist die leichte Handhabbarkeit, das reversible Anlegen, die Anpassbarkeit an den jeweiligen Anwender sowie der non-invasive Charakter. Oberflächenelektroden können jedoch verrutschen und müssen in der Regel präzise angeordnet werden, um eine ausreichende Signalqualität bereitzustellen. Weiterhin ist es möglich, über Implantate in dem Muskel elektrische Potentiale abzugreifen und diese über Schnittstellen oder drahtlos an Empfänger zu übermitteln, die dann die Signale in elektrischer Form an die Steuerungseinrichtung übermittelt. Ebenfalls ist es möglich, Drucksensoren an oder in den jeweiligen Muskeln zu positionieren. Durch die Muskelkontraktionen verändert sich die Form des Muskels, was wiederum zu Veränderungen des Druckes beispielsweise an einer Manschette, Spange oder einem Aufnahmeschaft für einen Stumpf, beispielsweise einem Oberschenkelschaft, führt. Die Sensoren können ebenfalls von außen an dem Muskel oder der Gliedmaße angelegt werden, beispielsweise über einen Gurt oder über ein elastisches Band mit einer Vorspannung versehen sein, um die jeweiligen Druckänderungen zu detektieren. Auch Veränderungen in der optischen Durchlässigkeit können zur Detektion von Muskelkontraktionen ausgenutzt werden. Es besteht die Möglichkeit, Spulen in einem Schaft unterzubringen und implantierte Elektroden in der Muskulatur mikroinvasiv einzupflanzen. Die implantierten Elektroden werden über die Spule oder die Spulen in dem Schaft induktiv mit Energie versorgt und übertragen die jeweils erfassten Daten drahtlos oder drahtgebunden über die Spule oder Spulen an eine Auswerteeinheit, die dann diese Daten der Steuerungseinrichtung übermittelt. Grundsätzlich wird die Muskelaktivierung über Elektroden, Sensoren oder Detektoren ermittelt. In einer Recheneinheit wird aus den elektrischen Signalen die Co-Kontraktion sowie deren Intensität und Dauer ermittelt und auf der Grundlage dieser Informationen wird der Steuerungs-Algorithmus angepasst.

Eine Variante der Erfindung sieht vor, dass die von der Erfassungseinrichtung, beispielsweise Oberflächenelektrodenanordnungen, Implantaten, Drucksensoreinrichtungen, optische Sensoreinrichtungen und/oder induktiv arbeitende Sensoreinrichtungen, detektierten Rohsignale in einer Pre-Processing-Einheit aufbereitet werden und erst die aufbereiteten Signale an die Steuerungseinrichtung übermittelt werden. Das Pre-Processing der Rohsignale von den jeweiligen Sensoren oder Detektoren erfolgt beispielsweise in einem Mikrokontroller oder in einer separaten Recheneinheit, die in der Nähe oder in einem baulichen Zusammenhang zu der Erfassungseinrichtung und den Detektoren steht. Beispielsweise kann die Pre-Processing-Einheit an einer der Komponenten, beispielsweise der proximalen Komponente, angeordnet sein und drahtlos oder über eine drahtgebundene Verbindung mit der Steuerungseinrichtung gekoppelt sein. Die Pre-Processing-Einheit übermittelt in einer Ausführungsform nur noch die für das Steuerungsverfahren relevanten Informationen der Steuerungseinrichtung, beispielsweise die Kokontraktionsintensität und die Kokontraktionsdauer oder nur das Signal, dass eine Kokontraktion vorliegt, um die eigentliche Steuerungseinheit, die gegebenenfalls auch noch andere Steuerungsaufgaben durchzuführen hat, nicht mit der Verarbeitung von Rohsignalen belasten zu müssen. Der Steuerungseinrichtung steht dadurch ein einfach zu verarbeitendes, aufbereitetes Signal zur Verfügung, das einfach in den bereits vorhandenen Steuerungsalgorithmus integriert werden kann. Die Erfassungseinrichtung und die Pre-Processing-Einheit können als Modul aufgebaut und als Zusatzbauteil für bereits vorhandene Steuerungen eingesetzt werden. Das Modul kann einen eigenen Energiespeicher aufweisen. Neben einer hohen Flexibilität durch den modularen Aufbau wird zudem keine negative Beeinflussung der Akkulaufzeit der bereits vorhandenen Steuerungseinrichtung bei einer zusätzlichen Funktionalität erreicht.

Als Kokontraktion werden nicht nur die Anspannung von Agonist und Antagonist, sondern allgemein die gleichzeitige Anspannung mehrerer Muskelgruppen, welche unterschiedliche oder unabhängige Funktionen erfüllen, angesehen. Ist beispielsweise ist einer der Muskeln eine Antagonist/Agonist-Paares nicht mehr vorhanden, kann die Aktivierung eines anderen Muskels oder einer andern Muskelgruppe als Antagonist des verbliebenen Muskels definiert werden. Ist zum Beispiel der Beinstrecker oder musculus quadriceps femoris vorhanden, der Beinbeuger oder musculus biceps femoris jedoch nicht, ist eine originäre Kokontraktion nicht möglich, so dass statt dessen die Kontraktion der Abduktoren und/oder der Bauchmuskulatur hinzugenommen werden kann, um eine Kokontraktionssignal für die Steuerung zu erhalten.

Die orthetische oder prothetische Einrichtung, die an einem Körper eines Anwenders anlegbar und daran festlegbar ist, mit einer Gelenkeinrichtung mit einer proximalen Komponente und einer distalen Komponente, die um eine Schwenkachse schwenkbar ineinander gelagert sind, zumindest einem verstellbaren Aktuator, der zwischen der proximalen Komponente und der distalen Komponente angeordnet ist und über die ein Bewegungswiderstand gegen eine Verschwenkung der proximalen Komponente relativ zu der distalen Komponente einstellbar ist, zumindest eine Erfassungseinrichtung zur Erfassung von Muskelkontraktionen sowie einer Steuerungseinrichtung, die mit der Erfassungseinrichtung und dem Aktuator gekoppelt ist, Signale von der Erfassungseinrichtung verarbeitet und in Abhängigkeit von den Signalen den Aktuator verstellt, sieht vor, dass die Erfassungseinrichtung zur Erfassung des Vorliegens von willkürlichen oder unwillkürlichen Muskelkokontraktionen und die Steuerungseinrichtung zur Durchführung des Verfahrens, wie es oben beschrieben ist, eingerichtet ist. Mit einer solchen orthetischen oder prothetischen Einrichtung, beispielsweise einer Orthese, einem Exoskelett oder einer Prothese, ist es möglich, willkürlich oder unwillkürlich das Widerstandsniveau des verstellbaren Aktuator situationsangepasst zu verändern und eine Überlagerung eines bereits vorhandenen Steuerungsprogrammes zu ermöglichen, um für einen Anwender auf einfache Art und Weise und intuitiv eine Anpassung an ein gesteigertes Sicherheitsbedürfnis zu erreichen. Das Gelenk wird steifer, da eine Relativverschwenkung von proximaler und distaler Komponente erschwert oder unterbunden wird, so dass der Nutzer eine bessere Kontrolle über die Einrichtung bekommt.

Die Erfassungseinrichtung kann als Oberflächenelektrodenanordnung, als Implantat, als Drucksensoreinrichtung, als optische Sensoreinrichtung und/oder als inaktiv arbeitende Sensoreinrichtung ausgebildet sein. Die Anordnung der Erfassungseinrichtung ist dabei so ausgebildet, dass Muskelkokontraktionen erfasst werden können. Dazu sind die jeweiligen Sensoren oder Detektoren an den entsprechenden Muskeln angeordnet oder mit ihnen gekoppelt, beispielsweise über Implantate entsprechend präpariert, sodass Muskelkontraktionen erfasst, Kokontraktionen festgestellt und entsprechende Veränderungen in dem Aktuator ausgeführt werden können.

Die Erfassungseinrichtung kann in einer Ausführungsform in der proximalen und/oder distalen Komponente integriert sein. Bei Prothesen ist beispielsweise ein Aufnahmeschaft für einen Stumpfteil der proximalen Komponente. Sensoren können fest installiert in dem Aufnahmeschaft und/oder in einem Prothesenliner angeordnet sein. Ebenso können Ausnehmungen oder Durchbrüche zur Positionierung von Elektroden oder anderen Sensoren in dem Schaft oder einer anderen Aufnahmeeinrichtung für ein Körperteil ausgebildet sein, sodass unterschiedliche Sensoren oder Sensoren auswechselbar und anpassbar an den jeweiligen Anwender an der proximalen Komponente befestigt werden können. Oberflächenelektroden könnten auf der Innenseite eines Schaftes einlaminiert oder dauerhaft befestigt sein. Eine separate Steuerungseinrichtung oder eine Pre-Processing-Einheit kann ebenfalls in dem Schaft oder in der proximalen Komponente integriert oder daran befestigt sein, gegebenenfalls mit einer eigenen Energieversorgung, um die Sensordaten hinsichtlich der jeweiligen Muskelkontraktionen aufzuarbeiten, auszuwerten und die aufbereiteten Daten der Steuerungseinrichtung zu übermitteln, die dann den Aktuator entsprechend mit Steuerungssignalen zur Veränderung des Bewegungswiderstandes versorgt.

In einer Ausführungsform der Erfindung ist an der orthetischen oder prothetischen Einrichtung zumindest ein Sensor zur Erfassung von Kräften, Winkeln, Positionen, Beschleunigungen und/oder Momenten angeordnet und mit der Steuerungseinrichtung gekoppelt. Der jeweilige Sensor kann als Dehnmessstreifen, Winkelsensor, Inertialwinkelsensor, Beschleunigungssensor oder Momentensensor ausgebildet sein. Wenn ein Sensor mehrere Größen oder Variablen erfassen kann, können diese Größen oder Variablen von nur einem einzigen Sensor erfasst werden, ansonsten sind für die Erfassung der unterschiedlichen Größen oder Variablen mehrere Sensoren notwendig. Über diesen Sensor oder über diese Sensoren und die damit erfassten Sensorwerte ist es möglich, Signale zu generieren, um über die Steuerungseinrichtung den Aktuator an den jeweiligen Bewegungszustand, Bewegungssituation, Belastungszustand und/oder Anwender anzupassen.

Die Erfassungseinrichtung kann mit einer Pre-Processing-Einheit gekoppelt sein, um Muskelkokontraktionsdaten zu identifizieren und aufbereitete Daten der Steuerungseinrichtung übermitteln zu können, damit diese schneller und effizienter arbeiten kann.

Die Erfassungseinrichtung und die Pre-Processing-Einheit können als gemeinsames Modul ausgebildet und an der orthetischen oder prothetischen Einrichtung oder an dem Anwender festlegbar sein, um mit der Steuerungseinrichtung gekoppelt zu werden. Dadurch ist es möglich, bereits bestehende orthetische oder prothetische Einrichtungen wir Orthesen, Exoskelette und Prothese mit der Erfassungseinrichtung und der Pre-Processing-Einheit auszurüsten und nachzurüsten.

Nachfolgend werden Ausführungsbeispiele der Erfindung anhand der beigefügten Figuren näher erläutert. Es zeigen:
- Figur 1 -: eine schematische Darstellung einer Protheseneinrichtung in Gestalt eines Prothesenkniegelenkes;
- Figur 2 -: eine Variante der Protheseneinrichtung gemäß Figur 1 ;
- Figur 3 -: eine schematische Darstellung einer Variante mit einer Pre-Processing-Einheit;
- Figur 4 -: eine schematische Darstellung einer Widerstandsänderung beim Gehen in der Ebene;
- Figur 5 -: eine schematische Darstellung einer Widerstandsänderung beim Abwärtsgehen;
- Figur 6 -: eine Darstellung von Situationen unwillkürlicher Kokontraktionen; sowie
- Figur 7 -: eine schematische Darstellung eines Widerstandsverlaufes.

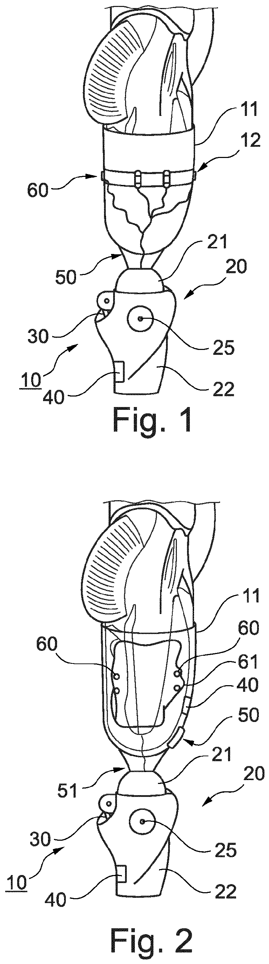

Figur 1 zeigt in einer schematischen Darstellung eine prothetische Einrichtung 10 in Gestalt eines Prothesenkniegelenkes mit einem Oberschenkelschaft 11, der an einem Patienten festgelegt ist. Der Oberschenkelschaft 11 kann über unterschiedliche Mechanismen an einem Oberschenkelstumpf festgelegt werden, beispielsweise über eine Saugschafttechnologie, eine Klemmung, eine osseointegrierte Festlegung oder auf andere Arten und Weisen. An dem distalen Ende des Oberschenkelschaftes 11 ist eine Gelenkeinrichtung 20 in Gestalt eines Prothesenkniegelenkes angeordnet, das eine proximale Komponente 21 mit Anschlussmitteln zum Befestigen an dem Oberschenkelschaft 11 aufweist. An der proximalen Komponente 21 ist eine distale Komponente 22 in Gestalt eines Unterschenkelteils schwenkbar um eine Schwenkachse 25 gelagert. Die Schwenkachse 25 ist in dem dargestellten Ausführungsbeispiel als Kniegelenksachse ausgebildet, bei anderen Anwendungen, beispielsweise bei einem Prothesenknöchelgelenk oder prothetischen oder ortethischen Einrichtungen an der oberen Extremität, sind entsprechend andere Gelenkachsen vorgesehen. Alternativ zu einer prothetischen Einrichtung 10 kann auch eine orthetische Einrichtung vorgesehen sein, bei der statt des Ersatzes einer Gliedmaße eine Unterstützung einer Gliedmaße mit einem noch vorhandenen natürlichen Gelenk erfolgt. Bei einer knieübergreifenden Orthese sind eine Oberschenkelschiene an einem Oberschenkel und eine Unterschenkelschiene an einem Unterschenkel festgelegt, beispielsweise durch Schnallen, Gurte oder Schalen. Die Oberschenkelschiene und die Unterschenkelschiene sind über ein Orthesenkniegelenk miteinander schwenkbar um eine Achse gekoppelt. Bei einer Knöchelorthese ist die proximale Komponente eine Unterschenkelschiene und die distale Komponente ein Fußteil, das im Bereich der natürlichen Knöchelgelenksachse über ein Gelenk schwenkbar daran angeordnet ist. Unter einer orthetischen Einrichtung werden auch Exoskelette verstanden.

Zwischen der proximalen Komponente 21 und der distalen Komponente 22 ist ein Aktuator 30 angeordnet, im dargestellten Ausführungsbeispiel ist der Aktuator 30 überwiegend an der distalen Komponente 22 angeordnet und in einem Gehäuse integriert. Der Aktuator 30 kann als Widerstandseinrichtung, beispielsweise als hydraulischer Dämpfer, pneumatischer Dämpfer oder magnetorheologischer Dämpfer ausgebildet sein. Ebenso ist es möglich, dass der Aktuator als ein motorischer Antrieb ausgebildet ist, beispielsweise als ein elektromotorischer Antrieb, hydraulischer Antrieb oder pneumatischer Antrieb. Auch bei einer Ausgestaltung eines Aktuators 30 als Antrieb kann dieser als eine Widerstandseinrichtung geschaltet werden, beispielsweise indem ein Elektromotor im Generatorbetrieb betrieben wird. An der proximalen Komponente 21 ist ein Ausleger angeordnet, der über eine Kolbenstange oder eine Schubstange mit der Widerstandseinrichtung oder dem Antrieb, also dem eigentlichen Aktuator 30, gekoppelt ist, wobei der Aktuator 30 an dem anderen Ende der Kolbenstange oder der Schubstange mit der distalen Komponente 22 verbunden ist.

Die proximale Komponente 21 führt zu der distalen Komponente 22 eine Verschwenkbewegung aus, in der dargestellten Position befindet sich die Gelenkeinrichtung 20 in einer maximal extendierten, also gestreckten Position. Von dieser Position aus erfolgt eine Flexionsbewegung, bei der die posteriore oder hintere Seite der proximalen Komponente 21 in Richtung auf die posteriore Seite der distalen Komponente 22 verschwenkt wird, sodass sich der Winkel auf der posterioren Seite zwischen den beiden Komponenten 21, 22 bei Flexion verringert und bei Extension, also bei einer Streckbewegung, vergrößert. Wenn sich der Flexionswinkel vergrößert, verringert sich den Kniewinkel. Um bei dem Gehen eine an die jeweils vorhandene Gehsituation angepasste Verschwenkbewegung sowohl in Flexionsrichtung als auch in Extensionsrichtung ausführen zu können, ist der Aktuator 30 einstellbar ausgebildet, um das Bewegungsverhalten bei der Flexion und/oder Extension zu beeinflussen. Durch eine Erhöhung des Flexionswiderstandes kann beispielsweise der maximale Flexionswinkel oder der minimale Kniewinkel eingestellt werden, ebenso kann bei einer Extensionsbewegung kurz vor Erreichen der maximal extendierten Stellung eine Widerstandserhöhung bereitgestellt werden, um eine harten Anschlag in die Extensionsstellung zu vermeiden. Ebenfalls ist es möglich, den Aktuator 30 als die Bewegung unterstützend auszugestalten, also als Antrieb, um eine Flexionsbewegung und/oder Extensionsbewegung einzuleiten oder zu unterstützen.

Um den Aktuator 30 einzustellen und den bereitgestellten Widerstand oder die bereitgestellte Unterstützung zu beeinflussen, ist der Aktuator 30 mit einer Steuerungseinrichtung 50 gekoppelt, die im dargestellten Ausführungsbeispiel der Figur 1 in dem distalen Endbereich des Prothesenschaftes 11 integriert ist. In der Steuerungseinrichtung sind Datenverarbeitungseinrichtungen, Anschlüsse, Kontaktflächen, Schnittstellen und/oder ein Energiespeicher angeordnet, um eingehende Sensordaten oder Daten einer Erfassungseinrichtung 60 zu verarbeiten. In dem dargestellten Ausführungsbeispiel der Figur 1 ist die Erfassungseinrichtung 60 als Oberflächenelektrodenanordnung ausgebildet, die über einen Gurt 12 an dem Oberschenkelschaft 11 und dem Oberschenkel befestigt ist. Die Oberflächenelektroden 60 erfassen myoelektrische Signale bei der Kontraktion der Oberschenkelmuskulatur und leiten diese über Kabel oder auch drahtlos zu der Steuerungseinrichtung 50. Zusätzlich ist zumindest ein Sensor 40 an der distalen Prothesenkomponente 22 angeordnet, um weitere Daten über die vorhandenen Belastungen, Kräfte, Momente, Winkelstellungen, Beschleunigungen und/oder Raumorientierungen zu erfassen und der Steuerungseinrichtung zu übermitteln. Sensoren 40 sind jedoch für die Ausführung des Verfahrens nicht erforderlich, aber als Ergänzung vorteilhaft.

Die Erfassungseinrichtung 60 mit einer Mehrzahl an umfänglich um den Stumpf angeordneten Oberflächenelektroden ermöglicht die Erfassung von Muskelkontraktionen über myoelektrische Signale. Alternativ zu einer Anordnung an einem Gurt 12 können die Oberflächenelektroden auch in dem Prothesenschaft 11 integriert sein. Die Erfassungseinrichtung 60 und die Oberflächenelektroden daran sind so angeordnet und ausgebildet, dass unterschiedliche Muskelgruppen hinsichtlich ihrer Aktivität erfasst werden können. Damit ist es möglich, Muskeln, die für gegenläufige Bewegungen zuständig sind oder daran beteiligt sind, beispielsweise der Quadriceps für die Extension und der Beinbizeps für die Flexion, zu erfassen und Muskelkokontraktionen, also gleichzeitige Anspannungen von Muskelgruppen, zu erfassen. Muskelkokontraktionen treten nicht nur bei antagonistisch wirkenden Muskeln oder Muskelgruppen auf. Kokontraktionen können auch dann entstehen und erfasst werden, wenn voneinander unabhängige Muskelgruppen angespannt werden, beispielsweise die Bauchmuskulatur zusammen mit dem Hüftbeuger oder dem Beinbeuger.

Eine Variante der prothetischen Einrichtung ist in der Figur 2 dargestellt, bei der zusätzlich zu einem Sensor 40 zur Erfassung von Zustandsinformationen, wie Kräften, Momenten, Winkelstellungen im Raum oder Beschleunigungen eine weitere Sensoreinrichtung 40 an dem Oberschenkelschaft 11 angeordnet ist. Auch diese Sensoreinrichtung 40 ist mit der Steuerungseinrichtung 50 gekoppelt, die in dem dargestellten Ausführungsbeispiel in dem Oberschenkelschaft 11 integriert ist. Die Steuerungseinrichtung 50 weist eine integrierte Energiespeichereinheit auf, beispielsweise ein Akkumulator oder eine Batterie. Die Erfassungseinrichtung 60 ist in dem dargestellten Ausführungsbeispiel als eine Vielzahl, also mindestens zwei implantierbare Elektroden ausgebildet, die an verschiedenen Stellen der Muskulatur des Oberschenkels implantiert sind. Über diese implantierten Elektroden 60 werden Muskelaktivitäten erfasst, entweder über die Erfassung eines elektrischen Potentials oder aber durch Erfassung von Drücken, Temperaturen, Durchflussgeschwindigkeiten, Beeinflussungen des optischen Verhaltens von Komponenten oder ähnliches. Die durch das Implantat erfassten Daten können über eine Spulenanordnung 61, die an dem Oberschenkelschaft 11 angeordnet oder darin integriert sind, aufgenommen und an die Steuerungseinrichtung 50 übermittelt werden. Ebenfalls ist es möglich, dass die Erfassungseinrichtung 60 als eine Kombination von implantierten Elementen und einer Spule 61 oder mehreren Spulen 61 ausgebildet ist, um durch bei Muskelkontraktionen auftretenden induktiven Effekten Muskelkontraktionen und insbesondere Muskelkokontraktionen zu erfassen.

An dem distalen Ende des Prothesenschaftes 11 ist ein Interface 51 zu der Gelenkeinrichtung 20 angeordnet, über das es möglich ist, die Sensordaten hinsichtlich der Muskelkokontraktionen und gegebenenfalls der Sensordaten aus dem Sensor 40 an dem Oberschenkelschaft 11 an den Aktuator 30 zu übertragen. Über das Interface 51 werden auch die Sensordaten des Sensors 40 an der distalen Prothesenkomponente 22 der Steuerungseinrichtung 50 übermittelt. Je nach Vorliegen von Muskelkokontraktionen wird dann der Aktuator 30 angesteuert und beeinflusst, beispielsweise durch Verstellung von Ventilen, Drosselquerschnitten, durch Aktivierung eines Elektromagnetes zum Beeinflussen magnetorheologischer Flüssigkeiten, durch Abbremsen eines Motors und/oder durch Aktivieren eines Antriebs.

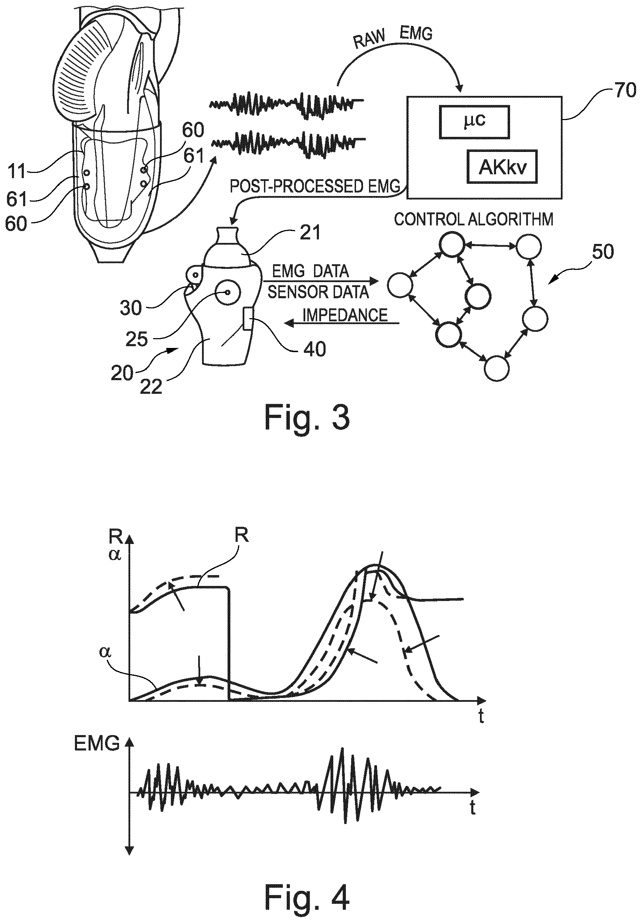

Figur 3 zeigt eine weitere Variante der Erfindung mit einem grundsätzlichen Aufbau, der dem der Figuren 1 und 2 entspricht. Der Oberschenkelschaft 11 ist von der proximalen Komponente 21 demontiert, ein mechanischer Anschluss des Oberschenkelschaftes 11 an der Gelenkeinrichtung 20 kann über den dargestellten Pyramidenadapter erfolgen. In dem Ausführungsbeispiel gemäß Figur 3 ist die Erfassungseinrichtung 60 wieder als implantierbare Elektrodenanordnung mit Spule 61 ausgebildet. Alternativ oder ergänzend ist es möglich, wie in der Figur 1 gezeigt, Oberflächenelektroden in Ausnehmungen innerhalb des Prothesenschaftes 11 an vordefinierten Stellen anzuordnen und über den Gurt 12 zu sichern oder auf der Innenseite des Prothesenschaftes 11 festzulegen. Über den Gurt 12, der als elastisches Band ausgebildet sein kann, werden die Elektroden an Positionen gehalten und mit einem ausreichenden Anpressdruck auf die Hautoberfläche gepresst. Der Oberschenkelschaft 11 selbst ist mit dem Prothesengelenk 20 elektrisch leitend verbunden, um die Daten der Erfassungseinrichtung 60 an die Gelenkeinrichtung 20 und insbesondere den Aktuator 30 zu übertragen.

Wie in der Figur 2 dargestellt, können die implantierten Elektroden 60 über die Spule 61 an oder in dem Oberschenkelschaft 11 induktiv mit Energie versorgt werden und sind so angeordnet, dass sie sich während der Anwendung innerhalb des Feldes der Spule 61 befinden. Die Datenübertragung erfolgt ebenfalls über die Spule 61. Gemäß der Ausführungsform der Figur 3 ist die Erfassungseinrichtung 60 zunächst mit einer Pre-Processing-Einheit 70 verbunden, der die Roh-Daten von den Elektroden 60 übermittelt werden. Statt über implantierte Elektroden 60 kann dies auch über Oberflächenelektroden gemäß Figur 1 erfolgen. Die Roh-Daten werden in der Pre-Processing-Einheit 70 aufbereitet, beispielsweise in einer Datenverarbeitungseinrichtung oder in einem Mikrocomputer, der in der Pre-Processing-Einheit 70 mit einer eigenen Energieversorgung angeordnet ist. Die Daten werden in einem Mikrocontroller, der direkt an dem Oberschenkelschaft 11 angeordnet sein kann, beispielsweise in dem distalen Anschlussstück des Oberschenkelschaftes 11 integriert sein kann, aufbereitet, um nur die relevanten oder aussagekräftigen Informationen weiterzuleiten. Aus der Pre-Processing-Unit 70 werden die aufbereiteten Daten dem Prothesenkniegelenk 20 übermittelt, beispielsweise über die Datenverbindung mittels der elektrischen Kopplung von Oberschenkelschaft 11 mit der proximalen Prothesenkomponente 21. Die verarbeiteten und aufbereiteten Roh-Daten der Erfassungseinrichtung 60 werden der Steuerungseinrichtung 50 übermittelt, über die dann die Impedanz oder der Widerstand bzw. der Antrieb des Aktuators 30 gesteuert wird. Der Steuerungseinrichtung 50 können auch Sensordaten der Sensoren 40 von dem Oberschenkel und/oder dem Unterteil übermittelt werden.

Die Pre-Processing-Einheit 70 kann gemeinsam mit der Erfassungseinrichtung 60 als ein Modul ausgebildet sein, das an einen bereits bestehenden Prothesenschaft 11 angeordnet werden kann. Bei implantierbaren Elektroden 60 sind die Elektroden 60 an beispielsweise die Spule 61 und die Pre-Processing-Einheit 70 angepasst und modular aufgebaut.

Die Erfassungseinrichtung 60, 61 und/oder die Pre-Processing-Einheit 70 können abschaltbar ausgebildet sein, sodass die Protheseneinrichtung 20 auch ohne die Steuerungseinrichtung 50 auf Basis der erfassten Muskelkokontraktionen funktionieren kann. Bevorzugt sind die Erfassungseinrichtung und/oder die Pre-Processing-Einheit 70 plug-and-play-fähig ausgebildet, sodass ein aufwendiges Abstimmungsverfahren zwischen den einzelnen Komponenten nicht mehr notwendig ist.

Das Grundprinzip der Steuerung ist bei allen Ausführungsbeispielen ähnlich, die Muskelaktivierung wird über die Erfassungseinrichtung 60, wie der Oberflächenelektroden oder implantierte Elektroden, gemessen und erfasst. In einer Recheneinheit, entweder in der Pre-Processing-Einheit 70 oder in der Steuerungseinrichtung 50, wird ermittelt, ob eine Muskelkokontraktion vorliegt. Es werden die Intensität und die Dauer der jeweiligen Muskelkontraktionen bestimmt. Auf Grundlage der gemessenen Muskelkontraktionen, wobei in der Steuerungseinrichtung 50 abgelegt werden kann, welche gleichzeitigen Muskelkontraktionen als Muskelkokontraktionen angesehen werden, wird über einen Steuerungsalgorithmus festgelegt, ob und wie der Aktuator 30 aktiviert oder deaktiviert wird, also ob eine Widerstandsveränderung oder eine Unterstützung einer Bewegung erfolgen soll. Die Zuordnung der Kontraktionssignale erfolgt bei dem Einrichten der Steuerung und Anpassung an den Anwender. In Abhängigkeit von der Position der jeweiligen Erfassungseinrichtung 60 kann definiert werden, welcher Muskel oder welche Muskelgruppe das entsprechende Kontraktionssignal verursacht.

Ist eine Pre-Processing-Einheit 70 der Steuerungseinrichtung 50 oder dem Aktuator 30 zugeordnet oder vorgeschaltet, werden der Gelenkeinrichtung 20 die aufbereiteten Daten, beispielsweise die Kokonktrationsintensität und die Dauer der Muskelkontraktionen, übermittelt, sodass der Gelenkeinrichtung 20 ein einfach zu verarbeitendes Signal zur Verfügung steht, das einfach in den Steuerungsablauf integriert werden kann. Dadurch wird der Steuerungsaufwand verringert und die Betriebsdauer der Protheseneinrichtung oder Ortheseneinrichtung wird verlängert, da die Signalaufbereitung keine negative Beeinflussung der Laufzeit der Gelenkeinrichtung 20 bei Einsatz und gegebenenfalls Nachrüstung mit der Erfassungseinrichtung 60 zur Folge hat. Insbesondere wenn die Pre-Processing-Einheit 70 nachrüstbar ist und über eine eigene Energieversorgung verfügt, wird die Betriebsdauer des Prothesengelenkes 20 nicht negativ beeinflusst. Gegebenenfalls kann durch einen einfachen Austausch der Pre-Processing-Einheit 70 mit eigener Energieversorgung die Gesamtbetriebsdauer der orthetischen oder prothetischen Einrichtung 10 verlängert werden.

In der Figur 4 ist eine schematische Darstellung des Verlaufes eines Bewegungswiderstandes R und eines Flexionswinkels α über der Zeit in Abhängigkeit von den erfassten Muskelkontraktionssignalen gezeigt. Dargestellt ist ein Schrittzyklus, beginnend mit dem Fersenstoß oder Heel Strike und endend mit dem Ende der Schwungphase kurz vor dem Aufsetzen der Ferse. Zunächst vergrößert sich der Flexionswinkel α, das heißt, dass sich der Kniewinkel verringert. Nach dem Aufsetzen der Ferse findet eine sogenannte Standphasenflexion statt, um nach dem Aufsetzen der Ferse eine Kraftdurchleitung durch ein gestrecktes Bein, beispielsweise eine gestreckte Prothese oder Orthese zu vermeiden. Dadurch wird die Stoßbewegung gedämpft und eine leichte Einbeugung des künstlichen Kniegelenkes ermöglicht. Nach dem vollständigen Aufsetzen des Fußes oder des Prothesenfußes erfolgt eine maximale Streckung während des sogenannten roll-over bis zum Ende der Standphase, bei der bereits eine Flexionsbewegung beginnt, deren Maximum in der Schwungphase erreicht wird, wenn das Knie maximal flektiert ist. Dann finden eine Bewegungsumkehr und eine Extension des Kniegelenkes statt. Dieses ist in der durchgezogenen Linie gezeigt. Der Widerstand R für das Gehen in der Ebene gegen eine Flexion steigt nach dem Heel Strike auf ein hohes Niveau an, um zu verhindern, dass das Kniegelenk ungewollt einbeugt. Auch nach Erreichen des roll-over und einer einsetzenden Standphasenextension bleibt der Widerstand R auf einem erhöhten Niveau, um dann bei einer einsetzenden Vorfußbelastung auf ein niedriges Niveau abzufallen, um eine Flexion zur Einleitung der Schwungphase zu ermöglichen. Im weiteren Verlauf des Gehens wird in der Schwungphase der Flexionswiderstand R angehoben, um die Flexion nicht zu weit auszuführen, damit am Ende der Schwungphase eine Streckung des Kniegelenkes erreicht werden kann. Kurz vor Erreichen der Bewegungsumkehr wird der Flexionswiderstand R nicht weiter erhöht und nach Erreichen des maximalen Flexionswinkels auf das Niveau verringert, das zur Dämpfung der Bewegung bei der Standphasenflexion benötigt wird. Ein erhöhtes Flexionsdämpfungsniveau am Ende der Schwungphase trägt zur Sicherheit des Anwenders bei, um bei einem Stolpern nicht ein ungewolltes Einbeugen des Kniegelenkes zu ermöglichen.

Wird, wie in der unteren Darstellung der Figur 4 dargestellt, eine Muskelkokontraktion detektiert, was an der hohen Amplitude in beiden Richtungen zu erkennen ist, wird der Flexionswiderstand R verändert, im dargestellten Ausführungsbeispiel angehoben, was durch die gestrichelte Linie gezeigt ist. Eine Kokontraktion kann beispielsweise unwillkürlich erfolgen, wenn ein Hindernis erfühlt oder gesehen oder eine glatte Oberfläche erkannt wird. Dann wird bereits beim Aufsetzen des Fußes oder des Prothesenfußes ein erhöhter Widerstand gegen ein Einbeugen des Kniegelenkes bereitgestellt, wodurch der maximal erreichbare Flexionswinkel α verringert wird, was ebenfalls durch die gestrichelte Linie dargestellt ist. Die Bewegungsbeeinflussung findet in dem dargestellten Ausführungsbeispiel durch eine stärkere und frühere Anhebung der Flexionsdämpfung in der Phase der Standphasenbeugung statt. Dadurch werden das Bewegungsausmaß, also der maximale Flexionswinkel α, reduziert und das Kniegelenk fühlt sich kompakter und steifer an. Bei einer Kokontraktion während der Schwungphase kann der Widerstand R ebenfalls früher und stärker angehoben werden, sodass der Flexionswinkel weniger schnell zunimmt und auch die maximale Schwungphasenflexion verringert wird. Das Kniegelenk pendelt weniger stark auf und kommt dadurch wesentlich früher und schneller in die volle Streckung. Dadurch wird eine erhöhte Sicherheit für den Anwender bereitgestellt.

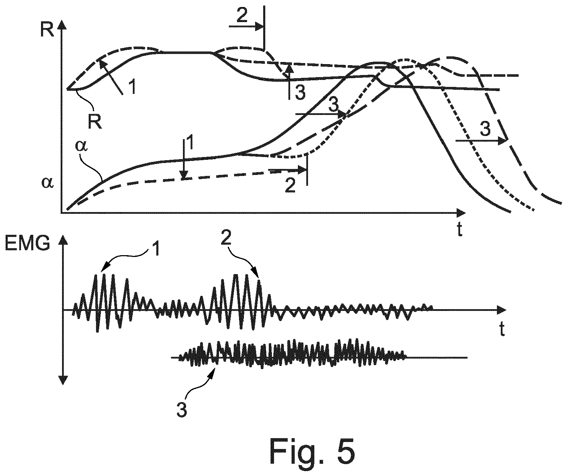

Eine Variante des Steuerungsverfahrens für das Bergabgehen ist in der Figur 5 gezeigt. Auch hier ist der Standardverlauf der Flexionsdämpfung R mit der durchgezogenen Linie dargestellt, der Kniewinkelverlauf α ohne Steuerung über Kokontraktionen ist ebenfalls mit der durchgezogenen Linie eingezeichnet. In dem Standard-Verfahren wird die Flexionsdämpfung R mit dem Einbeugen in der Standphase bis zur Sperrung der Flexion erhöht. Es stellt sich dann eine sogenannte Plateau-Phase ein, von der aus in der mittleren Standphase die Flexionsdämpfung R auf ein hohes Niveau reduziert wird, um ein weiteres Einbeugen zuzulassen, jedoch mit einem flacheren Anstieg des Flexionswinkels, um nach der Fersenablösung und Entlastung der Prothese eine Restschwungphase zu erlauben und sich auf den nächsten Heel Strike vorzubereiten. Werden Muskelkokontraktionen, wie in der unteren Darstellung der Figur 5 gezeigt, ermittelt, können diese Widerstände entsprechend früher angehoben oder auf einem höheren Niveau länger beibehalten werden, sodass das Kniegelenk später oder langsamer einbeugt und insgesamt ein höheres Dämpfungsniveau gegen eine Flexion bereitgestellt wird.

Neben den oben beschriebenen Ausführungen der Steuerung des Bewegungsverhaltens über veränderte Dämpfungen ist es möglich, das Bewegungsverhalten über andere Aktuatoren oder Widerstandseinrichtungen zu beeinflussen. Als Bewegungsbeeinflussung wird die Eigenschaft eines Systems angesehen, einer Bewegung eine Kraft entgegenzusetzen. Dies kann durch mechanische Elemente wie Federelemente mit einer definierten Steifigkeit und Nullpunkt, geschwindigkeitsproportional wirkende Dämpfungselemente, Reibelemente und/oder Massen geschehen. Ebenfalls kann die Bewegungsbeeinflussung über einen Motor, einen hydraulischen oder pneumatischen Dämpfer, Federelemente, piezoelektrische Elemente, hydraulische oder pneumatische Antriebe oder Kombinationen der genannten Elemente oder Komponenten erfolgen. Ein Höchstmaß an Einflussmöglichkeiten bieten aktive Antriebe, beispielsweise kann ein Elektromotor ein elastisches Verhalten simulieren und die gefühlte Trägheit des Gelenkes oder der prothetischen oder orthetischen Einrichtung beeinflussen und eine geschwindigkeitsabhängige Kraft erzeugen.

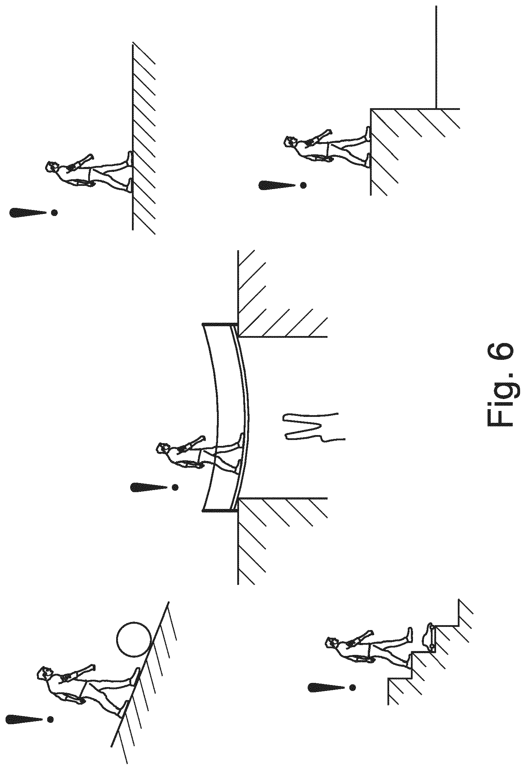

Situationsbeispiele für willkürliche oder unwillkürliche Kokontraktionen sind in der Figur 6 gezeigt, beispielsweise bei Auftreten eines Hindernisses, bei Erkennen einer Gefahr durch Tiere, Menschen oder Maschinen, bei glatten Oberflächen, bei plötzlich auftretenden Gefahrensituationen oder im Bereich von unsicheren oder gefährlichen Umgebungsbedingungen. Die reflexhafte Muskelkokontraktion oder auch die bewusste Muskelkokontraktion ermöglichen die Beeinflussung eines Steuerungsverfahrens durch Muskelaktivierung, wobei ein Grundmuster des Steuerungsverfahrens bevorzugt beibehalten wird, lediglich die Ausprägungen in der Stärke und der Dauer werden verändert oder beeinflusst. Insbesondere hat die Beeinflussung des Bewegungsverhaltens über Muskelkokontraktionen den Vorteil, dass diese sowohl bewusst als auch unbewusst erfolgen kann, wodurch die Reflexe des Menschen bei der Wahrnehmung der Umgebung vorteilhaft zur Steuerung der orthetischen oder prothetischen Einrichtung ausgenutzt werden können.

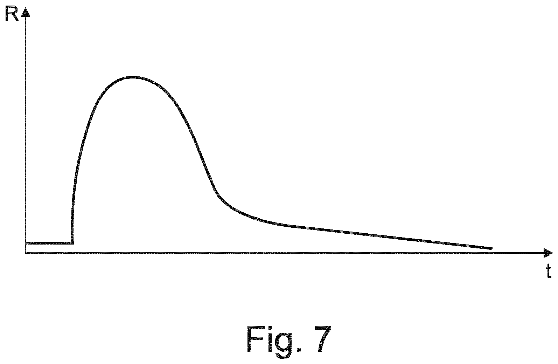

In der Figur 7 ist in einer schematischen Darstellung der Verlauf eines Widerstandes R gegenüber der Zeit aufgetragen. Wird beispielsweise eine Kokontraktion festgestellt, die es notwendig erscheinen lässt, den Widerstand gegen ein Einbeugen zu erhöhen, wird dieser Widerstand R sehr schnell erhöht, ausgehend von einem Basiswiderstand. Nach Erreichen eines Maximums des Widerstandes wird beispielsweise nach Nachlassen der Kokontraktion oder bei einer Detektion eines Umstandes, dass die Steuerung dazu veranlasst, den Widerstand wieder zu verringern, wird zunächst eine langsame Verringerung des Widerstandes R durchgeführt, sodass sich ein sanfter Abfall des Widerstandes über der Zeit ergibt. Dadurch wird verhindert, dass ein schneller Widerstandsabfall zu einem Verlust eines Gleichgewichts und zu unsicheren Situationen für den Nutzer führt.

## Patentansprüche

1. Verfahren zur Steuerung einer orthetischen oder prothetischen Einrichtung (10), die an einem Körper eines Anwenders anlegbar und daran festlegbar ist, mit
a. einer Gelenkeinrichtung (20) mit einer proximalen Komponente (21) und einer distalen Komponente (22), die um eine Schwenkachse (25) schwenkbar aneinander gelagert sind,
b. zumindest einem verstellbaren Aktuator (30), der zwischen der proximalen Komponente (21) und der distalen Komponente (22) angeordnet ist und über den ein Bewegungsverhalten bezüglich einer Verschwenkung der proximalen Komponente (21) relativ zu der distalen Komponenten (22) einstellbar ist,
c. zumindest einer Erfassungseinrichtung (60) zur Erfassung von Muskelkontraktionen sowie
d. einer Steuerungseinrichtung (50), die mit der Erfassungseinrichtung (60) und dem Aktuator (30) gekoppelt ist, Signale von der Erfassungseinrichtung (60) verarbeitet und in Abhängigkeit von den Signalen den Aktuator (30) verstellt,
**dadurch gekennzeichnet, dass**
die Erfassungseinrichtung (60) zur Erfassung von Muskelkokontraktionen eingerichtet ist und an einer Gliedmaße des Anwenders angeordnet und mit der Steuerungseinrichtung (50) gekoppelt wird, dass zumindest eine Muskelkokontraktion von der Erfassungseinrichtung (60) detektiert wird, dass ermittelt wird, ob eine Kokontraktion vorliegt und dass das Bewegungsverhalten durch den Aktuator (30) in Abhängigkeit von der detektierten Muskelkokontraktion verändert wird.

2. Verfahren nach Anspruch 1, **dadurch gekennzeichnet, dass** die Dauer und/oder Intensität der Muskelkokontraktion erfasst und in Abhängigkeit von der Dauer und/oder Intensität der Muskelkokontraktion das Bewegungsverhalten verändert wird.

3. Verfahren nach Anspruch 1 oder 2, **dadurch gekennzeichnet, dass** der Aktuator (30) einen Bewegungswiderstand gegen eine Verschwenkung bereitstellt und der Bewegungswiderstand bei Detektion einer Muskelkokontraktion vergrößert wird.

4. Verfahren nach einem der voranstehenden Ansprüche, **dadurch gekennzeichnet, dass** bei zunehmender Kokontraktionsintensität und/oder Kokontraktionsdauer der Bewegungswiderstand zunehmend vergrößert und/oder bei einer abnehmenden Kokontraktionsintensität und/oder Kokontraktionsdauer und/oder am Ende einer Kokontraktion und/oder bei Detektion einer anderen Kokontraktion und/oder durch einen aktiven Auslöser und/oder einen Sprachbefehl verringert wird.

5. Verfahren nach Anspruch 4, **dadurch gekennzeichnet, dass** der Bewegungswiderstand schneller erhöht als verringert wird.

6. Verfahren nach einem der voranstehenden Ansprüche, **dadurch gekennzeichnet, dass** die Veränderung des Bewegungsverhaltens einem voreingestellten Steuerungsprogramm überlagert wird.

7. Verfahren nach Anspruch 6, **dadurch gekennzeichnet, dass** die Veränderung die Größe der Bewegungsbeeinflussung und/oder die Dauer der Bewegungsbeeinflussung beeinflusst.

8. Verfahren nach einem der voranstehenden Ansprüche, **dadurch gekennzeichnet, dass** die Muskelkontraktionen von der Erfassungseinrichtung (60) als myoelektrische Signale, Drucksignale, induktiv erzeugte Signale und/oder opto-elektronisch erzeugte Signale der Steuerungseinrichtung (50) übermittelt werden.

9. Verfahren nach einem der voranstehenden Ansprüche, **dadurch gekennzeichnet, dass** zumindest ein Sensor (40) Kräfte, Winkel, Positionen, Beschleunigungen und/oder Momente an der orthetischen oder prothetischen Einrichtung (10) erfasst, Sensorsignale an die Steuerungseinrichtung (50) übermittelt und das Bewegungsverhalten aufgrund der Sensorsignale verändert wird.

10. Verfahren nach einem der voranstehenden Ansprüche, **dadurch gekennzeichnet, dass** die von der Erfassungseinrichtung (60) detektierten Rohsignale in einer Pre-Processing-Einheit (70) aufbereitet und in aufbereiteter Form an die Steuerungseinrichtung (50) übermittelt werden.

11. Verfahren nach einem der voranstehenden Ansprüche, **dadurch gekennzeichnet, dass** die erfassten Muskelkokontraktionen auf Plausibilität überprüft und Veränderungen des Bewegungsverhaltens bei nicht vorhandener Plausibilität abgelehnt oder rückgängig gemacht werden.

12. Orthetische oder prothetische Einrichtung (10), die an einem Körper eines Anwenders anlegbar und daran festlegbar ist, mit
a. einer Gelenkeinrichtung (20) mit einer proximalen Komponente (21) und einer distalen Komponente (22), die um eine Schwenkachse (25) schwenkbar aneinander gelagert sind,
b. zumindest einer verstellbaren Aktuator (30), der zwischen der proximalen Komponente (21) und der distalen Komponente (22) angeordnet ist und über den ein Bewegungsverhalten bezüglich einer Verschwenkung der proximalen Komponente (21) relativ zu der distalen Komponenten (22) einstellbar ist,
c. zumindest einer Erfassungseinrichtung (60) zur Erfassung von Muskelkontraktionen sowie
d. einer Steuerungseinrichtung (50), die mit der Erfassungseinrichtung (60) und dem Aktuator (30) gekoppelt ist, Signale von der Erfassungseinrichtung (60) verarbeitet und in Abhängigkeit von den Signalen den Aktuator (30) verstellt,
**dadurch gekennzeichnet, dass** die Erfassungseinrichtung (60) zur Erfassung des Vorliegens von willkürlichen oder unwillkürlichen Muskelkokontraktionen und die Steuerungseinrichtung (50) zur Durchführung des Verfahrens nach einem der voranstehenden Ansprüche eingerichtet ist.

13. Orthetische oder prothetische Einrichtung nach Anspruch 12, **dadurch gekennzeichnet, dass** die Erfassungseinrichtung (60) als Oberflächenelektrodenanordnung, als Implantat, als Drucksensoreinrichtung, als optische Sensoreinrichtung und/oder als induktiv arbeitende Sensoreinrichtung ausgebildet ist.

14. Orthetische oder prothetische Einrichtung nach Anspruch 12 oder 13, **dadurch gekennzeichnet, dass** die Erfassungseinrichtung (60) in der proximalen und/oder distalen Komponente (21, 22) integriert ist.

15. Orthetische oder prothetische Einrichtung nach einem der Ansprüche 12 bis 14, **dadurch gekennzeichnet, dass** zumindest ein Sensor (40) zur Erfassung von Kräften, Winkeln, Positionen, Beschleunigungen und/oder Momenten, der an der orthetischen oder prothetischen Einrichtung (10) angeordnet und mit der Steuerungseinrichtung (50) gekoppelt ist.

16. Orthetische oder prothetische Einrichtung nach einem der Ansprüche 12 bis 15, **dadurch gekennzeichnet, dass** die Erfassungseinrichtung (60) mit einer Pre-Processing-Einheit (70) gekoppelt ist.

17. Orthetische oder prothetische Einrichtung nach Anspruch 16, **dadurch gekennzeichnet, dass** die Erfassungseinrichtung (60) und die Pre-Processing-Einheit (70) als gemeinsames Modul ausgebildet sind.

18. Orthetische oder prothetische Einrichtung nach Anspruch 16 oder 17, **dadurch gekennzeichnet, dass** die Erfassungseinrichtung (60) und/oder die Pre-Processing-Einheit (70) abschaltbar und/oder plug-and-play-fähig ausgebildet sind.

19. Orthetische oder prothetische Einrichtung nach einem der Ansprüche 12 bis 18, **dadurch gekennzeichnet, dass** der Aktuator (30) als Widerstandseinrichtung oder Antrieb ausgebildet ist.

## Claims

1. A method for controlling an orthotic or prosthetic device (10) which can be placed on the body of a user and secured thereon, comprising
a. a joint device (20) with a proximal component (21) and a distal component (22), which are mounted pivotably on each other about a pivot axis (25),
b. at least one adjustable actuator (30), which is arranged between the proximal component (21) and the distal component (22) and via which a movement behavior with respect to a pivoting of the proximal component (21) relative to the distal component (22) is adjustable,
c. at least one detection device (60) for detecting muscle contractions, and
d. a control device (50) which is coupled to the detection device (60) and to the actuator (30), processes signals from the detection unit (60), and adjusts the actuator (30) according to the signals, **characterized in that**
the detection device (60) is designed for detecting muscle co-contractions and is arranged on a limb of the user and coupled to the control device (50), **in that** at least one muscle co-contraction is detected by the detection device (60), that it is determined whether there is a co-contraction and **in that** the movement behavior is changed by the actuator (30) according to the detected muscle co-contraction.

2. The method as claimed in claim 1, **characterized in that** the duration and/or intensity of the muscle co-contraction is detected, and the movement behavior is changed according to the duration and/or intensity of the muscle co-contraction.

3. The method as claimed in claim 1 or 2, **characterized in that** the actuator (30) provides a movement resistance against pivoting, and the movement resistance is increased when a muscle co-contraction is detected.

4. The method as claimed in one of the preceding claims, **characterized in that**, with an increasing co-contraction intensity and/or co-contraction duration, the movement resistance is increasingly heightened, and/or with a decreasing co-contraction intensity and/or co-contraction duration and/or at the end of a co-contraction and/or upon detection of another co-contraction, it is reduced by an active trigger and/or a voice command.

5. The method as claimed in claim 4, **characterized in that** the movement resistance is increased more quickly than it is reduced.

6. The method as claimed in one of the preceding claims, **characterized in that** the change in the movement behavior is superposed on a preset control program.

7. The method as claimed in claim 6, **characterized in that** the change influences the extent of the movement influence and/or the duration of the movement influence.

8. The method as claimed in one of the preceding claims, **characterized in that** the muscle contractions are transmitted from the detection device (60) to the control device (50) as myoelectric signals, pressure signals, inductively generated signals and/or opto-electronically generated signals.

9. The method as claimed in one of the preceding claims, **characterized in that** at least one sensor (40) detects forces, angles, positions, accelerations and/or moments on the orthotic or prosthetic device (10) and transmits sensor signals to the control device (50), and the movement behavior is changed on the basis of the sensor signals.

10. The method as claimed in one of the preceding claims, **characterized in that** the raw signals detected by the detection device (60) are processed in a pre-processing unit (70) and are transmitted in processed form to the control device (50).

11. The method as claimed in one of the preceding claims, **characterized in that** the detected muscle co-contractions are checked for plausibility and, in the absence of plausibility, changes in the movement behavior are rejected or reversed.

12. An orthotic or prosthetic device (10) which can be placed on the body of a user and secured thereon, comprising
a. a joint device (20) with a proximal component (21) and a distal component (22), which are mounted pivotably on each other about a pivot axis (25),
b. at least one adjustable actuator (30), which is arranged between the proximal component (21) and the distal component (22) and via which a movement behavior with respect to a pivoting of the proximal component (21) relative to the distal component (22) is adjustable,
c. at least one detection device (60) for detecting muscle contractions, and
d. a control device (50) which is coupled to the detection device (60) and to the actuator (30), processes signals from the detection unit (60), and adjusts the actuator (30) according to the signals,
**characterized in that** the detection device (60) is designed for detecting of the presence of voluntary or involuntary muscle co-contractions, and the control device (50) is designed for carrying out the method as claimed in one of the preceding claims.

13. The orthotic or prosthetic device as claimed in claim 12, **characterized in that** the detection device (60) is designed as a surface electrode arrangement, as an implant, as a pressure sensor device, as an optical sensor device and/or as an inductively operating sensor device.

14. The orthotic or prosthetic device as claimed in claim 12 or 13, **characterized in that** the detection device (60) is integrated in the proximal and/or distal component (21, 22).

15. The orthotic or prosthetic device as claimed in one of claims 12 through 14, **characterized in that** at least one sensor (40) for detecting forces, angles, positions, accelerations and/or moments is arranged on the orthotic or prosthetic device (10) and coupled to the control device (50).

16. The orthotic or prosthetic device as claimed in one of claims 12 through 15, **characterized in that** the detection device (60) is coupled to a pre-processing unit (70).

17. The orthotic or prosthetic device as claimed in claim 16, **characterized in that** the detection device (60) and the pre-processing unit (70) are designed as a common module.

18. The orthotic or prosthetic device as claimed in claim 16 or 17, **characterized in that** the detection device (60) and/or the pre-processing unit (70) can be switched off and/or designed to be plug-and-play capable.

19. The orthotic or prosthetic device as claimed in one of claims 12 through 18, **characterized in that** the actuator (30) is designed as a resistance device or drive.

## Revendications

1. Procédé de commande d'un dispositif orthopédique ou prothétique (10) qui peut être appliqué sur le corps d'un utilisateur et y être fixé, comprenant
a. un dispositif d'articulation (20) avec un composant proximal (21) et un composant distal (22) qui sont montés l'un sur l'autre de manière à pouvoir pivoter autour d'un axe de pivotement (25),
b. au moins un actionneur réglable (30) qui est disposé entre le composant proximal (21) et le composant distal (22) et qui permet de régler un comportement de mouvement par rapport à un pivotement du composant proximal (21) par rapport au composant distal (22),
c. au moins un dispositif de détection (60) pour détecter des contractions musculaires, ainsi que
d. un dispositif de commande (50) qui est couplé au dispositif de détection (60) et à l'actionneur (30), qui traite des signaux provenant du dispositif de détection (60) et qui déplace l'actionneur (30) en fonction des signaux,
**caractérisé en ce que**
le dispositif de détection (60) est conçu pour détecter des co-contractions musculaires et est disposé sur un membre de l'utilisateur et est couplé au dispositif de commande (50),
**en ce qu'**au moins une co-contraction musculaire est détectée par le dispositif de détection (60), et
**en ce que** l'on détermine s'il se présente une co-contraction, et
**en ce que** le comportement de mouvement est modifié par l'actionneur (30) en fonction de la co-contraction musculaire détectée.

2. Procédé selon la revendication 1,
**caractérisé en ce que** la durée et/ou l'intensité de la co-contraction musculaire est détectée, et le comportement de mouvement est modifié en fonction de la durée et/ou de l'intensité de la co-contraction musculaire.

3. Procédé selon la revendication 1 ou 2,
**caractérisé en ce que** l'actionneur (30) fournit une résistance au mouvement à l'encontre d'un pivotement, et la résistance au mouvement est augmentée lors de la détection d'une co-contraction musculaire.

4. Procédé selon l'une des revendications précédentes,
**caractérisé en ce que** la résistance au mouvement est augmentée de plus en plus lorsque l'intensité de la co-contraction et/ou la durée de la co-contraction augmente(nt), et/ou est diminuée lorsque l'intensité de la co-contraction et/ou la durée de la co-contraction diminue(nt), et/ou à la fin d'une co-contraction et/ou lors de la détection d'une autre co-contraction et/ou par un déclencheur actif et/ou par une commande vocale.

5. Procédé selon la revendication 4,
**caractérisé en ce que** la résistance au mouvement est augmentée plus rapidement qu'elle n'est diminuée.

6. Procédé selon l'une des revendications précédentes,
**caractérisé en ce que** la modification du comportement de mouvement est superposée à un programme de commande préréglé.

7. Procédé selon la revendication 6,
**caractérisé en ce que** la modification influence l'ampleur de l'influence sur le mouvement et/ou la durée de l'influence sur le mouvement.

8. Procédé selon l'une des revendications précédentes,
**caractérisé en ce que** les contractions musculaires sont transmises par le dispositif de détection (60) au dispositif de commande (50) sous forme de signaux myo-électriques, de signaux de pression, de signaux générés par voie inductive et/ou de signaux générés par voie optoélectronique.

9. Procédé selon l'une des revendications précédentes,
**caractérisé en ce qu'**au moins un capteur (40) détecte des forces, des angles, des positions, des accélérations et/ou des couples au niveau du dispositif orthopédique ou prothétique (10) et transmet des signaux de capteur au dispositif de commande (50), et le comportement de mouvement est modifié sur la base des signaux de capteur.

10. Procédé selon l'une des revendications précédentes,
**caractérisé en ce que** les signaux bruts détectés par le dispositif de détection (60) sont préparés dans une unité de prétraitement (70) et sont transmis sous forme préparée au dispositif de commande (50).

11. Procédé selon l'une des revendications précédentes,
**caractérisé en ce que** les co-contractions musculaires détectées sont contrôlées quant à leur plausibilité, et les modifications du comportement de mouvement sont refusées ou annulées en cas d'absence de plausibilité.

12. Dispositif orthopédique ou prothétique (10) pouvant être appliqué sur le corps d'un utilisateur et y être fixé, comportant
a. un dispositif d'articulation (20) avec un composant proximal (21) et un composant distal (22), qui sont montés l'un sur l'autre de manière à pouvoir pivoter autour d'un axe de pivotement (25),
b. au moins un actionneur réglable (30) qui est disposé entre le composant proximal (21) et le composant distal (22) et qui permet de régler un comportement de mouvement par rapport à un pivotement du composant proximal (21) par rapport au composant distal (22),
c. au moins un dispositif de détection (60) pour détecter des contractions musculaires, ainsi que
d. un dispositif de commande (50) qui est couplé au dispositif de détection (60) et à l'actionneur (30), qui traite des signaux provenant du dispositif de détection (60) et qui déplace l'actionneur (30) en fonction des signaux,
**caractérisé en ce que**
le dispositif de détection (60) est conçu pour détecter la présence de co-contractions musculaires arbitraires ou non, et
le dispositif de commande (50) est conçu pour mettre en oeuvre le procédé selon l'une des revendications précédentes.

13. Dispositif orthopédique ou prothétique selon la revendication 12,
**caractérisé en ce que** le dispositif de détection (60) est réalisé sous forme d'ensemble d'électrodes de surface, d'implant, de dispositif capteur de pression, de dispositif capteur optique et/ou de dispositif capteur à fonctionnement inductif.

14. Dispositif orthopédique ou prothétique selon la revendication 12 ou 13,
**caractérisé en ce que** le dispositif de détection (60) est intégré dans le composant proximal et/ou distal (21, 22).

15. Dispositif orthopédique ou prothétique selon l'une des revendications 12 à 14,
**caractérisé en ce qu'**il est prévu au moins un capteur (40) pour détecter des forces, des angles, des positions, des accélérations et/ou des couples, qui est disposé sur le dispositif orthopédique ou prothétique (10) et est couplé au dispositif de commande (50).

16. Dispositif orthopédique ou prothétique selon l'une des revendications 12 à 15,
**caractérisé en ce que** le dispositif de détection (60) est couplé à une unité de prétraitement (70).

17. Dispositif orthopédique ou prothétique selon la revendication 16,
**caractérisé en ce que** le dispositif de détection (60) et l'unité de prétraitement (70) sont réalisés sous forme de module commun.

18. Dispositif orthopédique ou prothétique selon la revendication 16 ou 17,
**caractérisé en ce que** le dispositif de détection (60) et/ou l'unité de prétraitement (70) sont réalisés de manière à pouvoir être déconnectés et/ou sont aptes au plug-and-play.

19. Dispositif orthopédique ou prothétique selon l'une des revendications 12 à 18,
**caractérisé en ce que** l'actionneur (30) est réalisé sous forme de dispositif de résistance ou d'entraînement.
